# EUROPEAN PATENT APPLICATION

(11) **EP 1 527 783 A1**
(43) Date of publication of application: **04.05.2005**
(21) Application number: 04105427.1
(22) Date of filing: 01.11.2004
(51) Int. Cl.: A61K 31/70, A61P 35/00, A61P 9/00

(54) **Iridoid-saccharide compound and method of using same**

(30) Priority: 31.10.2003 US 516334 P
(71) Applicant: HEALTH RESEARCH, INC., Buffalo, NY 14263 (US)
(72) Inventor: Pandey, Ravindra K., 14221, Williamsville (US); Dobhal, Mahabeer, University of Rajasthan Jaipur-302004 (IN); Graham, Andrew, L2H 2H6, Niagara Falls (CA); Oseroff, Allan, 14216, Buffalo (US)
(74) Representative: Weber, Dieter, Dr.

(57) **Abstract**

A method for treatment of hyperproliferative tissue which by exposing the hyperproliferative tissue to a sufficient quantity of a purified iridoid compound to inhibit its growth, where the iridoid compound includes a polysubstituted cyclopenta(c)dihydropyran where the cyclopenta ring is substituted at its 2' position with a ketofuryl group, where the numbering of the fused cyclopenta(c)dihydropyran ring structure includes heterocyclic oxygen, is counterclockwise and begins at the first carbon atom counterclockwise from the cyclopcnta ring so that oxygen is in the 2 position in the pyran ring. The invention also includes the mouse iridoid compounds.

## Description

### Background of the Invention

This invention relates to saccharide derivatives of iridoid compounds and further relates to their use in biological and medical methods such as treatment of hyperproliferative tissue including tumors and hyperproliferative blood vessels such as found in macular degeneration.

Most current treatments for cancer and other hyperproliferative tissue involve one or more of surgery, radiation and chemotherapy. These treatments have commonly had such serious and undesirable consequences for the patient that detractors have referred to these treatments as "cut, burn and poison." Sadly, there has been some basis for that position. Surgery is never comfortable for a patient. Radiation frequently destroys normal tissue near a tumor site with resulting pain, dysfunction or disfigurement. And chemotherapy, despite attempts to target it to cancer cells, often poisons the systems of a patient resulting in weakness, nausea, and organ and immune dysfunction.

It has been known that certain iridoid compounds are very biologically active. U.S. Patents 5,272,172; 5,374,653 and 5,459,160 describe certain iridoids as anti-hyperlipemia agents (reducing excess blood lipids) and as cholagogues (increasing bile flow). The iridoids described in these U.S. Patents have relatively simple structures generally without pendant heterocyclic or saccharide moieties. U.S. Patents 4,401,825 and 4,401,825 describe iridoid structures, again having relatively simple pendant groups, as intermediates for prosanoids/protaglandins. U.S. Patent 5,929,038 describes an iridoid compound having inhibitory effects upon hepatitis B. The compounds of U.S. 5,929,038 may have a glucose moiety on the pyran ring but have no other pendant heterocylic structures. U.S. Patents 6,022,888 and 6,222,478 describe iridoids may have vascularization inhibition properties. The compounds are broadly claimed even though the breadth of the data is not commensurate with the broad scope of the claims. No compounds having a furan ring directly connected to the cylopenta ring are described nor are saccharide derivative pendant groups disclosed. U.K. Patent Application 2,104,383 discloses cyclopentadihydropyrans. The compounds in the UK Patent Application are described as having anti-fouling properties, but requires that the substituent at the 3' position of furane ring be an ethylene group substituted at the -CH₂- carbon atom with acetyl or a 1-keto, 2-ene, 3-(p-substituted phenyl) propylene group.

Plumeria genus belongs to the family Apocynaceae. Some species of this genus are widely used for the treatment of various ailments in traditional folklore medicine as bitter tonic, expectorant, purgative as well as in the treatment of skin diseases. The bark of some of the species has been found to be biologically active as diuretic, antipsychotic, and antitumor agents and as an inhibitor of human immune deficiency virus type-1 (HIV-1). *Plumeria* species have formally been investigated for isolation of a variety of iridoids and triterpenoids, which exhibited algicidal, antibacterial, cytotoxic and plant growth inhibitor activity.

### Brief Description of the Invention

In accordance with the invention a method is provided for treatment of hyperproliferative tissue which comprises exposing the hyperproliferative tissue to a sufficient quantity of a purified iridoid compound to inhibit its growth. The iridoid compounds for use in accordance with the invention may be purified from natural sources or may be made synthetically. The iridoid compounds suitable for use in accordance with the invention comprise polysubstituted cyclopenta(c)dihydropyrans where the cyclopenta ring is substituted at its 2' position with a ketofuryl group where the numbering of the fused cyclopenta(c)dihydropyran ring structure including heterocyclic oxygen, is counterclockwise and begins at the first carbon atom counterclockwise from the cyclopenta ring so that oxygen is in the 2 position in the pyran ring. Within this group of compounds, the invention also includes such compounds substituted with a mono or polysaccharide moiety especially those compounds where the pyran ring is substituted at its 1 position by an -O-saccharide substituent, i.e. where the -O-saccharide substituent is an -O-polysaccharide or an -O-monosaccharide. The most common saccharide substituent is a glucoside moiety.

More specifically the invention includes compounds of the formula: 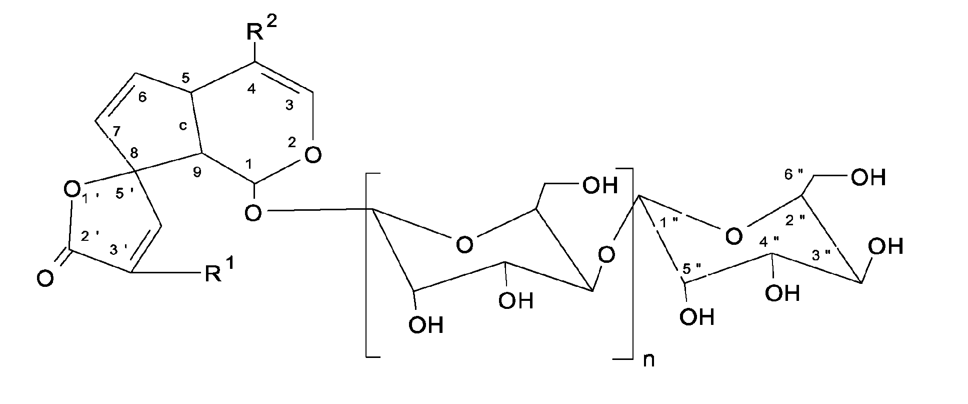 where R¹ and R² are independently lower alkenyl of 1-8 carbon atoms, lower alkyl of 1-8 carbon atoms, carboxy, carboxy C1-C8 lower alkyl (including salts and esters thereof), C1-C8 lower alkyl carboxy (including salts and esters thereof), hydroxy, C1-C8 hydroxy lower alkyl, lower alkylene-lower alkyl ether, lower alkyl amino, lower alkylene alkyl amine or C1-C8 alkylene alkyl ketone and n is an integer of 0 through 3, and their use for treating hyperproliferative tissue.

### Brief Description of the Drawings

Figure 1a shows probable structural formulas 1 (α-amyrine) and formula 2 (α-amyrine acetate), major components isolated from the bark of *Plumeria bicolor* having five fused rings.

Figure 1b shows a probable structural formula 3, for a major component isolated from the bark of *Plumeria bicolor* having an iridoid (plumeride) structure with a pendant saccharide ring.

Figure 1c shows a probable structural formula 4, for a major component isolated from the bark of *Plumeria bicolor* having an iridoid structure with a pendant propenene group.

Figure 1d shows a probable structural formula 5, for a major component isolated from the bark of *Plumeria bicolor* having an iridoid (plumeride) structure that is a sterioisomer of structural formula 4.

Figure 2 shows a bar graph of percent *in vitro* survival (cytotoxicity) of Colo-26 tumor cells at variable concentrations of compound 3 versus controls. Vehicle: 1% Tween 80 in 5% dextrose solution.

Figure 3 shows a graph of in vivo cytotoxicity of compound 3 against Colo-26 tumors at variable concentrations in mice.

Figure 4 shows *in vivo* tumor growth inhibition activity of compound 3 in mice bearing Colo-26 tumors.

### Detailed Description of the Invention

"Hyperproliferative tissue", as used herein means tissue characterized by abnormal and commonly accelerated growth. Examples of such hyperproliferative tissue are malignant and non-malignant tumors and hypervascularization such as is found in macular degeneration.

In a preferred embodiment the invention the invention comprises a purified iridoid compound having the formula: 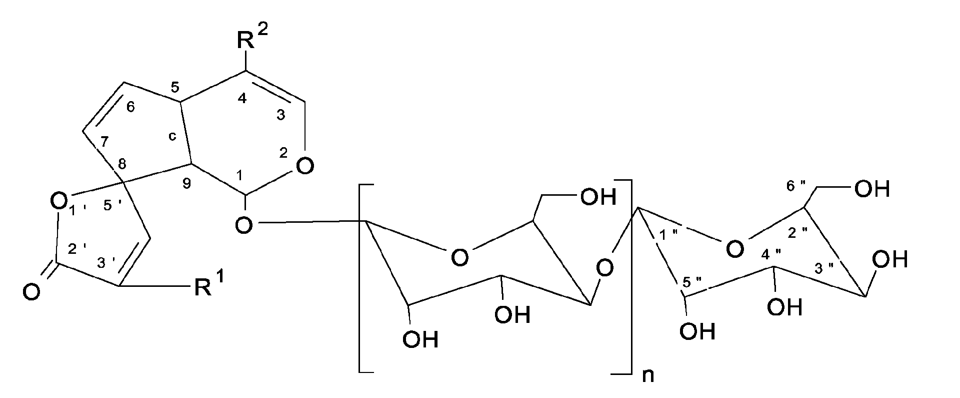 where R¹ and R² are independently lower alkenyl of 1-8 carbon atoms, lower alkyl of 1-8 carbon atoms, carboxy, carboxy C1-C8 lower alkyl (including salts and esters thereof), C1-C8 lower alkyl carboxy (including salts and esters thereof), hydroxy, C1-C8 hydroxy lower alkyl, lower alkylene-lower alkyl ether, lower alkyl amino, lower alkylene alkyl amine or C1-C8 alkylene alkyl ketone and n is an integer of 0 through 3. n is often 0 and R² is often -CO₂R⁴ where R⁴ is C1-C8 lower alkyl or C1-C8 hydroxy alkyl. R⁴ is commonly -CH₃ or -CH(OH)CH₃.

In a further preferred embodiment, the iridoid compound of the invention may have the formula: 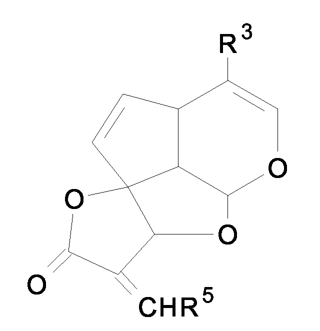 where R³ is independently lower alkenyl of 1-8 carbon atoms, lower alkyl of 1-8 carbon atoms, carboxy, carboxy C1-C8 lower alkyl (including salts and esters thereof), C1-C8 lower alkyl carboxy (including salts and esters thereof), hydroxy, C1-C8 lower alkyl hydroxy, lower alkylene-lower alkyl ether, or lower alkyl amino. R³ is commonly -CO₂R⁵ where R⁵ is lower alkyl. R⁵ is usually methyl.

Even more specifically the invention includes an iridoid-saccharide plumieride compound of the invention from the bark of the plant *Plumeria bicolor* of the plant family Apocynaceae, commonly known as frangipani, its method of use to treat hyperproliferative tissue and its method of preparation by repeated column chromatography of the methanolic extract of the bark to obtain a mixture of four compounds followed by elution of the column with petroleum ether to remove a white solid containing two of the compounds and subsequent elution with a 1:3 mixture of petroleum ether and benzene to obtain the purified iridoid-saccharide plumieride compound.

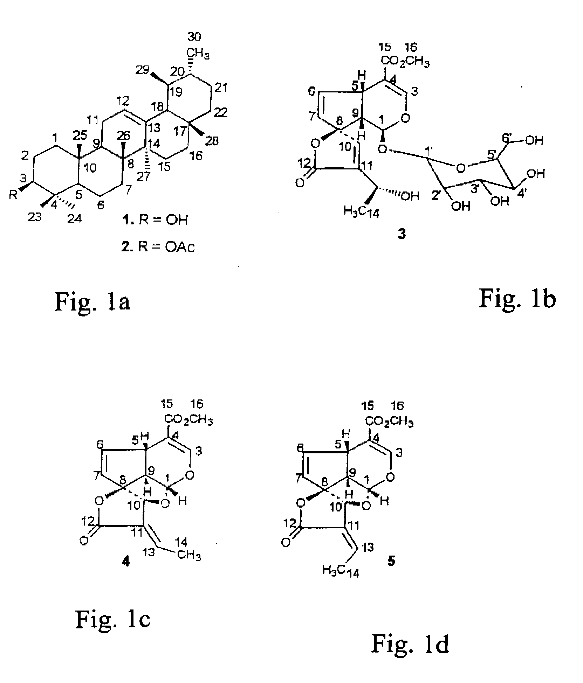

More specifically, the bark of *Plumeria bicolor* was collected from the campus of Rajasthan University, Jaipur, India. The authenticity was confirmed by comparing with the herbarium of the department of Botany, University of Rajathan, Jaipur. The residue obtained from the methanolic extract of the bark of the plant was purified by repeated column chromatography and afforded a mixture of four compounds. The mixture was purified into pure products on eluting the column with solvents of variable polarity. The product obtained by eluting with petroleum ether as a solvent gave a white solid, which was further purified by preparative TLC into two bands. On the basis of spectroscopic analyses, the faster moving band was identified as α-amyrin acetate, whereas the more polar component was characterized as α-amyrin. The compounds obtained on further elution with a mixture of petroleum ether/benzene (1:3) and pure benzene afforded two monoterpene in 0.81% and 0.56% yield respectively. Their structures were confirmed by NMR and mass spectrometry analyses as plumieride 3 and plumericin 4 respectively (Figures 1b and 1c). On the basis of mass spectrometry analysis, the molecular formula of the slow moving band was determined as C₂₁H₂₆0₁₂ (M⁺ 470). In the IR spectrum, the absorption observed at 3200 cm-1 (broad band) suggested the presence of the hydroxyl group(s) 1745-1755 cm⁻¹ (α,β-unsaturated lactone >C=O stretching). 1600, 1620 cm-1 (C=C stretching). In ¹H NMR spectrum, a doublet observed at 1.40 (J=2Hz) for three protons was assigned for a methyl group present at C-14. A singlet at 3.30 and a doublet at 3.95 were assigned for protons C-9 and C-5 respectively. A sharp singlet at 3.75 clearly suggested the presence of carboxymethyl group. The hydroxyl group present at C-13 appeared as a doublet at 4.62 (*J*=2.4Hz). A doublet observed at 4.75 was assigned for the proton present at C-13. The presence of a doublet at 5.55 (*J*=1.6Hz) was found to be a characteristic for the proton of a β-D-glucopyranose at C-1 carbon atom. The resonances observed at 5.46 and 6.45 (*J*=2Hz) were assigned to C-7 and C-6 proton respectively (a characteristic of a Δ⁶-iridoid-based system). Other olefinic protons present at C-3 and C-10 appeared as two singlets at 7.45 and 7.85 respectively. The appearance of two singlets at 3.25 and 3.30 were assigned for protons present at C-2' and C-4' respectively. A doublet observed at 3.95 was assigned for the proton at C-5'. A double triplet appeared at 4.42 was assigned for protons at C-6'. The glucosyl proton at C-1' observed as a triplet at 5.12 (*J*=2.4Hz).

In the ¹³C NMR spectrum, the six olefinic carbon atoms were observed at 150.70 (C-3), 108.50 (C-4), 128.05 (C-6), 140.16 (C-7), 148.06 (C-10) and 136.94 (C-11). The peak observed at 92.79were assigned to the anomeric carbon atom and the presence of a glucose moiety (98.60 (C-1'); 76.14 (C-2'); 78.1 (C-3'); 72.7 (C-4'); 78.1(C-5') and 60.61 (C-6') was also confirmed. On the basis of these results, the structure was assigned as plumieride 3.

The ¹H NMR spectrum (expressed in δ ppm) of the faster moving band was similar to3 except the resonances for the glucose moiety and a singlet observed for one proton at 7.85 (position-10) were absent. Instead, a new singlet was observed at 5.05 with a significant downfield shift generally that observed in substituted tetrahydrofuran ring systems. The presence of ethylidene group (=CH-CH₃) at position-11 (instead of (1'-hydroxyethyl) group present in plumieride 3) was confirmed by the presence of the resonances at 7.15 (dd) and 2.14 (d) integrating for one and three protons respectively. On the basis of the NMR and mass spectrometry analyses, the structure of the product was confirmed as plumericin 4. The possibility of isoplumericin 5, a geometrical isomer was ruled out due to the absence of the resonances at 6.73 and 2.25 reported for C-13 and C-14 protons for such system.

A literature survey revealed that compounds 3 and 4 had previously been isolated from various different species of *Apocynaceae,* however, the literature does not appear to recognize the antitumor activity of these compounds in pure form and does not indicate that such compounds could be purified from *Plumeria bicolor.*

*In vitro* and *in vivo* biological activity:

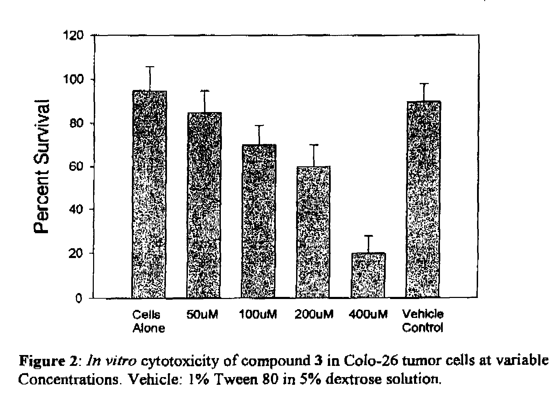

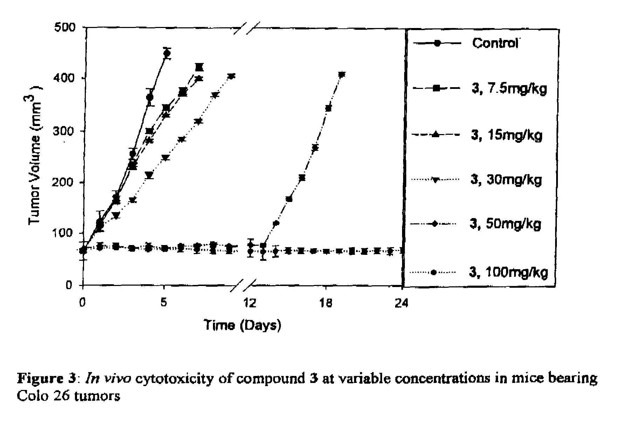

All the plant products isolated from the bark of *Plumeria bicolor* were insoluble in water and were dissolved in 1% Tween 80 in 5% dextrose solution. Among these analogs, plumeiride 3 produced good cytotoxicity producing a lethal dose 60%(LD60) with 200µM, and an LD20 with 400µM C-17in colo-26 tumor cells. Under these doses, the formulation alone did not produce any cytotoxicity (Figure 2). Plumieride 3 was then evaluated for *in vivo* efficacy in mice (6 mice/group) bearing colo-26 tumors at variable concentrations. As can be seen from Figure 3, the untreated control mice reached a size of 400 mm³ in approximately 5 days. A significant tumor growth delay was observed in mice that received compound 3. Mice that were injected daily with 50 mg/kg/mouse showed no increase in tumor volume for 12 days. This response was doubled to 24 days when the dose was increased to 100 mg/kg/mouse (Figure 3).

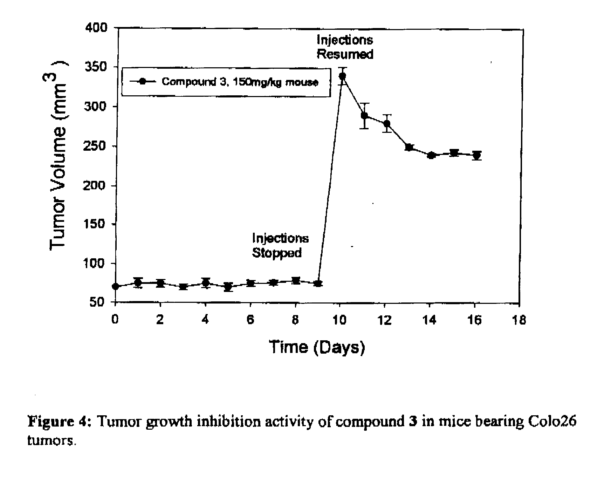

In another set of experiment, mice (6/group) were injected with compound 3 at a slightly higher dose (150 mg/kg/mouse) and produced a complete tumor inhibition as long as the drug was injected. On day 9, the drug administration was stopped and tumors were allowed to grow until they reached the size of approximately 350 mm³ in volume. The drug injection was resumed (150 mg/kg/mouse) daily for another 7 days. As can be seen from Figure 4, the tumor volume regressed to almost half the size when the injections were re-initiated, and then plateaued. In preliminary screening, no visible toxicity as well as a change in body weight or daily habits was observed.

In summary, our present study presents the characterization and biological evaluation of a series of pentacyclic triterpenoids 1, 2 and iridoid analog 3 isolated for the first time from the bark of *Plumeria bicolor.* Among these components, plumieride 3 containing a glucose moiety was found to be most effective. A significant tumor growth inhibition indicates a possibility of antiangiogenic characteristic of this class of compounds. In order to confirm our hypothesis, the detailed biological studies with this and a series of the related modified structures are currently in progress.

¹H and ¹³C NMR spectra of all the compounds were recorded on a 300MHz spectrometer in CDCl₃ solutions. The chemical shifts are expressed in part per million downfield from TMS. Thin-layer chromatography was done on a Merck coated plates 60F₂₅₄. Commercial grade solvents were used for extraction without purification. The mass spectrometry analyses were performed at the Biopolymer Facility, RPCI, Buffalo.

Shade dried and powdered bark (1 Kg.) was exhaustively extracted with methanol on a steam bath for 48 hrs. The extract after filtration was concentrated under reduced pressure. The extract (21g) so obtained was re-dissolved in minimum quantity of methanol and precipitated by adding CH3CN. The soluble (non-fatty) portion after concentration under reduced pressure afforded a gray-green semi solid (14.6g), which was chromatographed over Silica gel column and eluted with solvents of increasing polarity. Initial elution with petroleum ether gave a product that showed the presence of two components and was separated by preparative TLC using petroleum ether-acetone (2:3) as a mobile phase.

The fast moving band was identified as α-amyrin acetate 2, mp 220-210°C. ¹H NMR (CSCl₃, δ ppm) 0.75 (s, 3H, C-25), 0.80 (s, 3H, C-26), 0.85 (s, 3H, C-28), 0.95 (s, 3H, C-24),1.05 (s, 6H, C-23, C-27), 1.25 (s, 6H, C-29, C-30), 2.05 (s, 3H, OCOCH₃), 4.50 (m, 1H, C-3), 5.15 (t, 1H, C-12) and 1.30-1.95 (23H). Mass calculated for C₃₂H₅₂O₂: 468. Found: (M⁺ 468).

The slow moving band was characterized as α-amyrin 1, mp. 182-83°C (reported mp 183-184°C). ¹H NMR 0.78 (s, 3H, C-25), 0.81 (s, 3H, C-26), 0.84 (s, 3H, C-28), 0.94 (s, 3H, C-24), 1.05 (s, 6H, C-23, C-27), 1.28 (s, 6H, C-29 and C-30), 3.25 (m, 1H, C-3). Mass calculated for C₃₀H₅₀O: 426. Found: (M⁺ 426).

Further elution of the column with petroleumether-benzene (1:3) gave plumieride 3, which was crystallized from acetone mp. 225-228°C, ¹H NMR 1.40 (d, 3H, *J* 2.0Hz, C-14), 3.30 (s, 1H, C-9), 3.95 (d, 1H, C-5), 3.75 (s, 3H, COOCH₃), 4.62 (d, *J* 2.4, OH at C-13), 4.75 (d, 1H, C-13), 5.58 (d, *J,* 1.6Hz, C-1'), 5.48, 6.45 (each d, *J*, 2.0Hz, C-7, C-6, characteristic for Δ⁶ iridoids), 7.45, 7.85 (each s, C-3, C-10) ¹³C NMR (CDCl₃+DMSO-d₆), 92.79 C-1), 150.77 (C-3), 108.50 (C-4), 40.33 (C-5), 128.05 (C-6), 140.16 (C-7), 95.58 (C-8), 48.55 (C-9), 148.06 (C-10), 136.94 (C-11), 170.40 (C-12), 61.74(C-13), 21.90 (C-14), 166.02 (C-15), 50.85 (C-16), 98.60 (C-1'), 76.14 (C-2'), 78.11 (C-3'), 72.73 (C-4'), 78.11 (C-5'), 60.61 (C-6'). Mass calculated for C₂₁H₂₆O₁₂: 470. Found: (M⁺ 470).

On eluting the column with benzene plumericin 4 was obtained as white solid which was crystallized from acetone as light pale powder, mp. 210-110°C, ¹H NMR (CDCl₃) 2.04 (d 3H, C-14), 3.45 (m, 1H, C-9), 3.75 (s, 3H, C-16), 3.85 (dt, 1H, C-5), 5.05(s, 1H, C-10), 5.55 (m, 1H, C-6), 6.00 (dd, 1H, C-7), 7.15 (dd, 1H, C-13), 7.45 (s, 1H, C-3), ¹³C NMR (CDCl₃) 102.25(C-1), 151.85 (C-3), 103.36 (C-4), 37.95 (C-5), 141.18 (C-6), 141.00 (C-7), 108.15 (C-8), 52.93(C-9), 80.25 (C-10), 125.93 (C-11), 166.98 (C-12), 145.05 (C-13), 15.85 (C-14), 165.32 (C-15), 56.86 (C-16). Mass calculated for C₁₅H₁₄O₆: 290. Found: (M⁺ 290), 218 (base peak, 100%).

## Claims

1. The use of a purified iridoid compound to prepare a composition for treatment of hyperproliferative tissue which by exposing the hyperproliferative tissue to a sufficient quantity of the compound inhibit its growth, said preparation comprising adding physiologically acceptable excipients to an iridoid compound comprising a polysubstituted cyclopenta(c)dihydropyran where the cyclopenta ring is substituted at its 2' position with a ketofuryl group, where the numbering of the fused cyclopenta(c)dihydropyran ring structure includes heterocyclic oxygen, is counterclockwise and begins at the first carbon atom counterclockwise from the cyclopenta ring so that oxygen is in the 2 position in the pyran ring.

2. The use of claim 1 where the pyran ring is substituted at its 1 position by an -O-saccharide substituent.

3. The use of claim 2 wherein the -O-saccharide substituent is an -O-polysaccharide.

4. The use of claim 2 wherein the -O-saccharide substituent is an -O-monosaccharide.

5. An iridoid-saccharide plumieride compound purified from the bark of the plant *Plumeria bicolor* of the plant family Apocynaceae, commonly known as frangipani, by repeated column chromatography of the methanolic extract of the bark to obtain a mixture of compounds followed by elution of the column with petroleum ether to remove a white solid and subsequent elution with a 1:3 mixture of petroleum ether and benzene to obtain the purified iridoid-saccharide plumieride compound.

6. A purified iridoid compound having the formula: 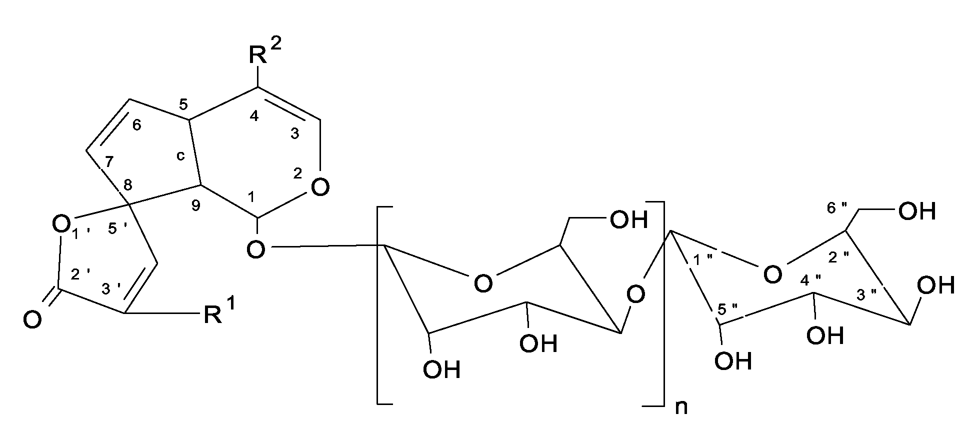 where R¹ and R² are independently lower alkenyl of 1-8 carbon atoms, lower alkyl of 1-8 carbon atoms, carboxy, carboxy C1-C8 lower alkyl (including salts and esters thereof), C1-C8 lower alkyl carboxy (including salts and esters thereof), hydroxy, C1-C8 hydroxy lower alkyl, lower alkylene-lower alkyl ether, lower alkyl amino, lower alkylene alkyl amine, C1-C8 alkylene alkyl ketone or -CH(OX)CH3 where X=H or an alkyl group and n is an integer of 0 through 3.

7. The compound of claim 6 where R¹ is =CHCH₃.

8. The compound of claim 6 wherein n is 0.

9. The compound of claim 8 wherein R² is -CO₂R⁴ where R⁴ is C1-C8.

10. The compound of claim 9 wherein R⁴ is -CH₃

11. The compound of claim 10 wherein R⁴ is -CH(OH)CH₃.

12. The compound of claim 10 wherein R² is -CONHR⁶ where R⁶ is a C1-C8 alkyl, aromatic carbohydrate or amino acid moiety.

13. An iridoid compound having the formula: 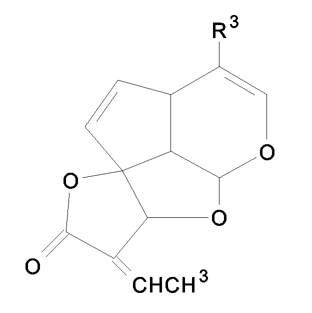 where R³ is lower alkenyl of 1-8 carbon atoms, lower alkyl of 1-8 carbon atoms, carboxy, carboxy C1-C8 lower alkyl (including salts and esters thereof), C1-C8 lower alkyl carboxy (including salts and esters thereof), hydroxy, C1-C8 lower alkyl hydroxy, lower alkylene-lower alkyl ether, or lower alkyl amino.

14. The compound of claim 9 wherein R³ is -CO₂R⁵ or 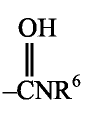 where R⁵ is C1-C8 lower alkyl and R⁶ is a C1-C8 lower alkyl, aromatic, amino acid or carbohydrate moiety.

15. The compound of claim 10 wherein R⁵ is methyl.
